# EUROPEAN PATENT SPECIFICATION

(11) **EP 0 629 136 B1**
(45) Date of publication and mention of the grant of the patent: **10.06.1998**
(21) Application number: 93904034.1
(22) Date of filing: 03.03.1993
(51) Int. Cl.: A61M 15/00

(54) **DISPOSABLE INHALER II**
WEGWERFINHALATOR
INHALATEUR II JETABLE

(30) Priority: 04.03.1992 EP 92850046
(43) Date of publication of application: 21.12.1994
(73) Proprietor: Astra Aktiebolag, 151 85 Södertälje (SE)
(72) Inventor: KÄLLSTRAND, Göran, S-237 00 Bjärred (SE); NILSSON, Per-Gunnar, S-212 14 Malmö (SE)
(74) Representative: Linderoth, Margareta
(86) International application number: PCT/EP93/00473
(87) International publication number: WO 93/17728

(56) References cited:
- EP-A- 0 404 454
- WO-A-89/01348
- WO-A-92/04069
- US-A- 4 265 236
- US-A- 5 042 472

## Description

### Technical field of the invention.

The present invention relates to a disposable breath-actuated inhaler comprising a tubular housing having at least two parts forming an air flow path being open at both ends, one end forming an air inlet and one end forming an air outlet, said housing comprising a compartment for storing a pharmaceutical powder to be inhaled, said housing is being shaped with a constriction adjacent the compartment in order to obtain a turbulent air stream at the constriction upon inhalation thereby lifting the powder out from the indentation and mixing it into the air stream.

### Background of the invention.

Disposable, breath-actuated inhalers of the kind described above are for instance disclosed in WO 89/01348, US-A-4,265,236, EP-A-0404454, US-A-5 042 472 and WO-A-9204069.

EP-A-0404454 discloses a disposable, breath-actuated inhaler comprising a chamber for a pharmaceutical powder, said chamber being provided with an air inlet and with an air outlet. The air inlet and outlet are covered by a common cover. The powder is disposed loosely in said comparatively large chamber which means that the powder not necessarily will be located at that location at which the air flow is most efficient.

US-A-4,265,236 discloses a tubular disposable, breath-actuated inhaler comprising a flexible tube, the ends of which normally being sealingly inserted into each other. This kind of seal will not necessarily be moisture-proof. There furthermore is a risk that some amount of the powder may fall out of the inhaler when the ends of the tube are pulled apart.

WO 89/01348, in the embodiment most of interest here, discloses a tubular, disposable inhaler which is sealed in both ends by means of twist-off caps. The pharmaceutical powder is loosely disposed in the inhaler and, as in the other inhalers described above, there is a risk that some powder is lost when the inhaler is opened.

US-A-5 042 472 discloses a breath-actuated inhaler having a pharmaceutical active powder provided in a compartment which is includes the inhalation inlet and outlet as well as the inhalation chamber. The powder is provided loose in the chamber in a manner which is similar to what is shown in EP-A-0 404 454 as mentioned above. The disadvantages with a device like this has already been discussed above.

WO-A-9 204 069 describes an inhaler of the type described in the upper part of claim 1 and which constitutes the background to the present invention. This is a disposable breath-actuated inhaler having a tubular housing defining the air flow path. An air inlet and an air outlet is provided at each end of the tubular housing. A pharmaceutically active powder is placed in a compartment located adjacent the air inlet. The powder compartment is formed as an indentation or recess. However, it has been shown that the powder provided in the indentation will during handling and storage be compacted to large powder aggregates. During inhalation this could lead to that not all powder is lifted up into the air stream by the inhalation air force. The result of this could be a significant inaccuracy of the inhaled doses.

The objects of the invention are to provide a disposable inhaler of the kind described above in which the dose of pharmaceutical powder can be determined accurately and in which the pharmaceutical powder can be stored hermetically sealed and moisture-proof. The dose delivered by different specimens of the same inhaler should generally be constant. The inhaler finally should be easy to prepare for use and easy to use as well as being easy and cheap to manufacture.

### Brief description of the inventive concept.

The above objects are achieved in that the disposable inhaler is provided with the features set forth in the appended main claim. Advantageous embodiments are set forth in the dependent claims.

### Brief description of the appended drawings.

- Fig 1: shows a perspective view of an inhaler according to the invention,
- Fig 2: shows a perspective view of an inhaler according to Fig 1 but showing the two main parts of the inhaler in an unassembled state,
- Figs 3A - 3C: show different stages in the opening of the powder compartment of the inhaler of Fig 1,
- Fig 4: shows an end view of the air inlet of the inhaler in Fig 1,
- Figs 5 - 6: show different possible embodiments of the constriction adjacent the powder compartment.

### Detailed description of a preferred embodiment of the invention.

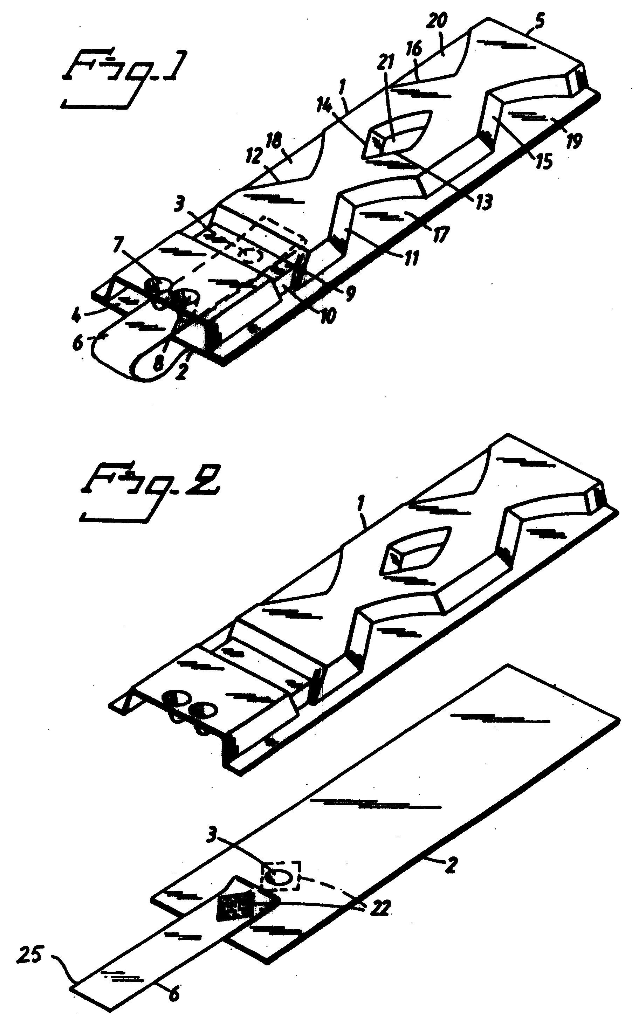

A preferred embodiment of the invention is disclosed in Figs 1 - 4. In Fig 1 the inhaler can be seen in a fully assembled condition and ready for use. As can be seen, the inhaler essentially comprises two elongate main parts, an upper part 1 which is made of a moulded sheet of plastic material and a lower part 2 preferably made of aluminium foil laminated with plastic. The upper part 1 is U-shaped with a substantially rectangular shape. The width of the upper part is several times the height. The lower part is generally flat and the two parts thus form a tubular housing defining an air conduit or air flow path with an air inlet 4 and an air outlet 5. A part-spherical depression, recess or indentation 3 indicated with a dashed line is located close to the air inlet 4. The recess 3 forms a powder compartment and is covered by a tape 6 which preferably is made of aluminium foil, also laminated with plastic. The recess 3 is provided with one or several small through-holes 24 in the bottom. The hole or holes 24 should be large enough to allow the entry of air into the recess from the underside of the lower part, but sufficiently small to prevent any part of the powder from falling out through the hole(s).

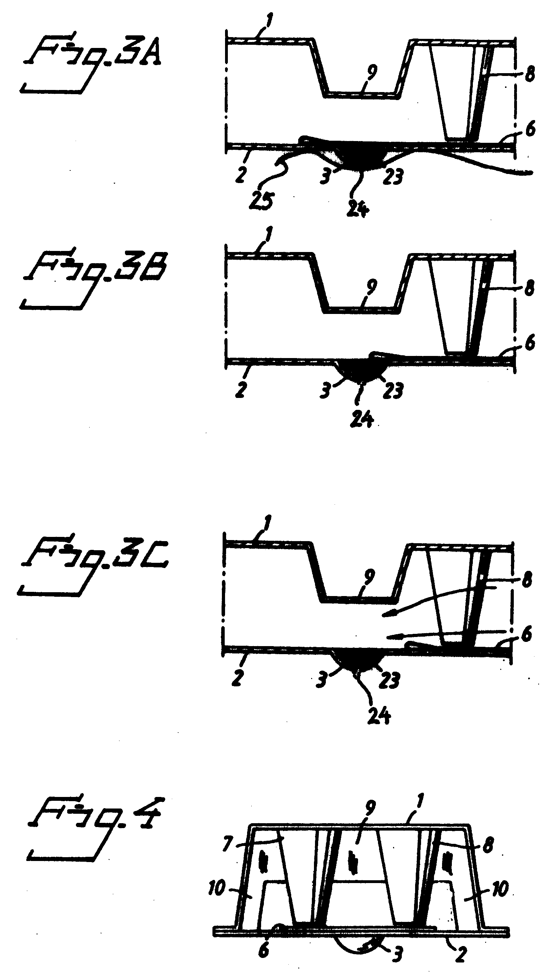

As indicated, the end of the part of the tape 6 covering the recess 3 is located between the recess 3 and the air inlet 4. The tape is attached to the lower part 2 around the powder compartment by means of a relatively weak weld 22 which can be seen in Fig 2. The end of the tape is attached by a comparatively large and thus stronger weld in front of the compartment, as seen in the intended direction of the air flow. The free part of the tape 6 is bent backwards over the recess 3 and extends out through the air inlet 4. In this particular embodiment, the free part of the tape is guided and held by two conical projections 7,8 extending downwards from the upper part 1. The free part of the tape may be bent in a loop to the underside of the lower part 2 and attached to the lower part all around the recess 3 by a relatively weak weld, thus sealing the hole 24 and the recess 3. The tape should be sufficiently long to extend past the recess 3, thus forming a tab 25 to serve as a grip for tearing the tape away.

A constriction in the flow path in the form of a ridge 9 oriented perpendicularly relative to the direction of the flow path is located above the powder compartment. The ridge is formed as a depression 9 in the upper part 1. The ridge is delimited at each end by an abutment 10.

The inhaler is further provided with deaggregation means after the powder compartment, as seen in the direction of the intended air flow through the inhaler. These deaggregation means comprise a number of oblique planar surfaces which are oriented at an angle of about 30° relative to the longitudinal direction of the inhaler, it surprisingly having been found that the most efficient angle of a planar surface relative to the air flow direction for disintegrating powder agglomerations is about 30°. Since the air flow will be deflected to some extent by the planar surface, the flow direction will not coincide fully with the longitudinal direction, but the above angle has been chosen as being the best compromise. The angle can however be varied between 20° and 50° with a preferred range of 25° - 35°.

The planar surfaces are oriented generally perpendicularly relative to the lower part 2, or at least as perpendicularly as the method of manufacturing the inhaler allows. The planar surfaces are located in such a way that their projections onto a cross-sectional plane substantially cover the entire cross-section of the inhaler. The projections preferably should overlap to some extent in order to ensure that any larger particles or agglomerations entrained in the air flow will impact on at least one such surface. In the preferred embodiment the planar surfaces 11, 12, 13, 14, 15, 16 are located on the upstream ends of two pairs of indentions 17, 18; 19, 20, formed into the sides of the upper part 1 and on the upstream end of a central depression 21 located between said indentations forming an island in the flow path. The downstream ends of said indentations and said depression taper in the direction of the air flow and have a smooth, rounded shape in order to obtain good aerodynamic conditions without any areas where the powder entrained in the air flow could settle.

The two main parts of the inhaler are shown separated in Fig 2. Apart from the details shown in Fig 1, the powder compartment 3 is shown opened, the tape 6 having been pulled outwardly through the air inlet. The shape of the (broken) weld 22 can be seen on the tape 6 and around the powder compartment 3. As can be seen, the shape of the weld has been chosen to be the perimeter of a square oriented with one diagonal parallel with the longitudinal extent of the inhaler. This means that the disengagement of the tape from the lower part 2 will be facilitated since the tearing action will both start and end at a corner. Since the weld holding the inner end of the tape is broad and strong, the user will feel when the compartment has been uncovered by means of the increased resistance.

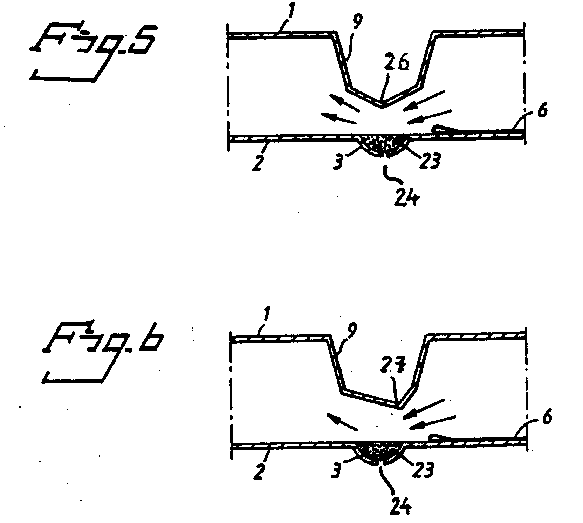

Figs 3A - 3C show different stages in the opening of the powder compartment 3 by pulling the tape 6, thus exposing the powder 23. Fig 3A shows the tape 6 attached around the recess 3 on the under side of the lower part, thus covering the hole 24 whilst leaving a tearing tab 25.

The end view in shown Fig 4 more clearly illustrates the inter-relationship between the upper part 1, the lower part 2, the powder compartment 3, the tape 6, the conical projections 7, 8, the ridge 9 and the abutments 10.

When the inhaler is to be used, the inhaler is held more or less horizontal with the flat half 2 facing downwards. The free end of the tape 6 is pulled by means of the tab 25, thus exposing the hole 24 and the powder in the powder compartment 3. The two conical projections 7, 8 will hold the tape 6 flat against the lower part 2 and thus prevent the tape from occluding the constriction in front of the powder compartment. The user then inserts the air outlet into the mouth and inhales through the inhaler. The resultant air flow through the inhaler will become very turbulent in the region of the constriction and the pharmaceutical powder will be lifted out of the powder compartment and mixed with the air flow. Any particles adhering to the tape may also be entrained with the air flow since the part of the tape originally covering the powder compartment also will lie directly in the flow path. By admitting air into the bottom of the recess 3, the through-hole 24 will counteract any subpressures in the bottom of the recess which might impede the lifting of the powder out of the recess.

Tests have shown that the dose leaving a typical powder compartment (e.g. about 0.5 mg) located at a constriction having an area of about 10 - 12 mm² will remain essentially constant at air flow rates varying from 30 l/min to 60 l/min.

The powder-laden air will then flow from the constriction to the deaggregation means. The angle of attack of the oblique surfaces will entail that the lighter particles, i. e. the particles within the respirable range, < 6µm, will be deflected from the surface without sticking thereto and thus mainly will follow the air flow, whereas the heaver particles and agglomerates will impact on and rebound from the planar surfaces and in this way be broken up into smaller particles. As mentioned above, an angle of about 30° may be optimal.

In this case tests again have shown that the percentage of particles within the respirable range in the dose to be inhaled will remain substantially constant at air flow rates ranging from 30 l/min to 60 l/min.

That the dose of respirable particles remains substantially constant over a wide range of air flows is important in order to minimize the difference between patients with different inhalation capacities.

It should be noted that the tubular shape of the inhaler makes it possible to mount a rubber ball or similar over the air inlet. By the means thereof the powder could be ejected from the inhaler into the throat of the patient in synchronization with the breathing of the patient by a helper if the patient should be incapable to use the inhaler by himself.

As mentioned above, the lower part 2 of the inhaler as well as the tape 6 preferably are made of aluminium foil laminated or coated with a suitable plastic. The aluminium will ensure the desired protection against moisture whereas the plastic will ensure that the tape can be welded to the lower part and that the lower part can be welded to the upper part. The lower part may for instance consist of a aluminium foil having a thickness of 45 µ which on one side is covered with a layer of oriented polyamide which is 25 µ thick and on the other side is covered by a layer of polypropene which is 60 µ thick. The upper part is preferably made of polypropene being 300 or 400 µ thick. The upper part can be transparent so that the user can see if the dose has been ejected from the powder compartment.

The tape may be made of a laminate having a "peel"-effect comprising polyester, aluminium and a layer comprising a polymer mixture of polybuten and polypropene.

The choice of material in the inhaler should be adapted to the drug to be used. The above materials have been chosen with a specific drug (budesonide) in mind, these materials releasing a dose of this drug more easily.

The composition of the pharmaceutical powder of course is quite optional and the powder may for instance comprise a pure active substance, a mixture of different active substances or a mixture of active substance(s) with adjuvant(s). It should be pointed out that the scope of choice of drugs is widened considerably due to the moisture-proof containment of the drug in the powder compartment.

The inhaler may be manufactured in the following way. A series of half-spherical indentations are formed in a strip of laminated aluminium foil in order to shape powder compartments. If the indentations are to be provided with through-holes, these are also formed at this stage. The indentations are filled with drugs and are topped off by means of scrapers, which will ensure a substantially uniform size of the different doses. An aluminium tape laminated with plastic is then welded over each indentation and around the indentation on the outside of the lower part.

The lower parts are then welded to upper parts and the strip is cut to form individual inhalers which are ready for packaging and use. The upper parts are moulded from sheets of plastic. In the moulding procedure care should be taken to ensure that the side walls of the upper part are as perpendicular as possible relative to the upper side in order to ensure an air flow which is as uniform as possible throughout the entire cross-section of the inhaler. The function of the abutments 10 primarily are to prevent that the ridge forming the constriction is distorted during the welding process.

### Possible modifications of the invention.

The invention of course can be modified in many ways within the scope of the appended claims.

Thus the ridge 9 forming the constriction can be designed in different ways in order to enhance the lifting action of the air flow on the powder. Some examples thereof can be found in Figs 5 - 6.

Figs 5 and 6 illustrate two alternative embodiments wherein the ridge has been provided with an edge 26 resp 27 oriented along the longitudinal extent of the ridge and which also will direct some air flow more directly into the powder compartment.

These embodiments will however require a higher degree of precision in the manufacturing in order to obtain the desired effect than the embodiment described above and will therefore be more difficult to manufacture.

The ridge 9 forming the constriction has been illustrated as being generally trapezoid in cross-section and as being generally rectilinear in longitudinal section. It should however be pointed out that the constriction may be shaped in many different ways within the scope of the appended claims.

The powder compartment can of course have another shape than a half-spherical shape and may for instance be elliptical, the minor axis thereof being parallel with the direction of the air flow, or may be otherwise trough-shaped. It is of course also possible to have several indentations, for instance if it is desired to increase the dose in an exactly defined way. Two separate tapes furthermore can be used to seal the recess 3 respectively the hole(s) 24.

The projections 7,8 can be shaped otherwise than conically and may for instance be shaped such that they direct a greater part of the air flow more directly past the powder compartment. They also could be integrated with the abutments 10.

The tape 6 could also be arranged in such a manner that it can be removed entirely from the lower part 2. In this case the projections 7,8 are not needed, at least not for the purpose of guiding and holding the tape.

The deaggregation means can be designed in other ways than in the form of planar surfaces oriented at an angle of about 20° - 50° relative to the direction of the air flow. This angle can of course also be varied outside this range and the surfaces do not necessarily have to be planar.

The material in the lower part and the tape does not necessarily have to comprise aluminium and may be any plastic material having the necessary impermeability and stiffness or having been treated to have these properties.

It is also conceivable to make the inhaler from a single sheet which is rolled or folded after having been moulded in an appropriate way.

## Claims

1. Disposable breath-actuated inhaler comprising a tubular housing having two parts (1, 2) forming an air flow path being open at both ends, one end forming an air inlet (4) and one end forming an air outlet (5), said housing comprising a compartment (3) for storing a pharmaceutical powder to be inhaled, said housing is being shaped with a constriction(9) adjacent the compartment (3) in order to obtain a turbulent air stream at the constriction (9) upon inhalation thereby lifting the powder out from the compartment (3) and mixing it into the air stream, wherein the compartment is provided as an indentation (3) being in contact with the ambient air through at least one through-hole (24) provided in the bottom of said indentation, the indentation (3) being located adjacent the air inlet (4), said indentation (3) and the at least one through-hole (24) is covered by a foil (6) sealing the indentation and the at least one through-hole in an airtight way, said foil being removable from the indentation and the at least one through-hole from outside the housing.

2. Inhaler according to claim 1,
**characterised** in that the at least two parts of the housing are formed as a moulded upper part (1) and a generally flat lower part (2) in which the powder indentation is provided, said upper and lower parts (1, 2) being joined together along their longitudinal sides.

3. Inhaler according to claim 1 or 2,
**characterised** in that the upper part (1) is moulded from a sheet of plastics material.

4. Inhaler according to claim 3,
**characterised** in that said constriction (9) is formed as a depression in the upper side of the said upper part (1) which is oriented transversally relative to the longitudinal extent of the tubular housing and located above the indentation (3) formed in the lower part of the housing.

5. Inhaler according to any one of the preceding claims,
**characterised** in that the lower part (2) is made of aluminium foil laminated with plastics.

6. Inhaler according to any one of the preceding claims,
**characterised** in that the foil (6) is in the form of a tape having one free end extending out through the air inlet (4), said tape being attached around edges of the indentation (3) by means of relatively weak welds(22).

7. Inhaler according to claim 6,
**characterised** in that the inner end of the foil (6) is attached to the lower part (2) between the air inlet (4) and the indentation (3) the foil extending past, and being attached around, the indentation (3), the foil then being bent backwards about so as to extend out through the air inlet (4).

8. Inhaler according to claim 7,
**characterised** in that the weak welds (22) form a point facing downstream.

9. Inhaler according to any one of claims 6 to 8,
**characterised** in that projections (7, 8) extend downwards from the upper part (1) between the indentation (3) and the air inlet (4) for holding the foil (6) against the lower part (2) of the housing.

10. Inhaler according to any one of the preceding claims,
**characterised** in that deaggregation means (11, 12, 13, 14, 15, 16) are located in the air flow path between the indentation (3) and the air outlet (5).

11. Inhaler according to claim 10,
**characterised** in that said deaggregation means (11, 12, 13, 14, 15, 16) comprise planar surfaces oriented 20° - 40°, preferably about 30°, relative to the longitudinal direction of the tubular housing, said surfaces being disposed generally perpendicular to a plane through the longitudinal axis of the tubular housing, a projection of the planar surfaces onto a cross-section of the housing substantially covering said cross-section.

## Patentansprüche

1. Durch Atmung aktivierter Wegwerfinhalator, umfassend ein röhrenförmiges Gehäuse mit zwei Teilen (1, 2), die einen an beiden Enden offenen Luftströmungsweg bilden, wobei ein Ende einen Lufteinlaß (4) und das andere Ende einen Luftauslaß (5) bildet, wobei das Gehäuse ein Kompartiment (3) zur Aufbewahrung eines pharmazeutischen Pulvers zur Inhalation aufweist und mit einer Verengung (9) neben dem Kompartiment (3) ausgebildet ist, um bei Inhalation eine turbulente Luftströmung an der Verengung (9) zu erzielen, durch die das Pulver aus dem Kompartiment (3) angehoben und mit dem Luftstrom vermischt wird, worin das Kompartiment als Vertiefung (3), die mit der Umgebungsluft über mindestens ein Durchgangsloch (24) im Boden der Vertiefung in Verbindung steht, ausgebildet ist, wobei die Vertiefung (3) sich neben dem Lufteinlaß (4) befindet, und die Vertiefung (3) und das mindestens eine Durchgangsloch (24) von einer die Vertiefung und das mindestens eine Durchgangsloch abdichtenden Folie (6) luftdicht abgedeckt sind, wobei die Folie von der Vertiefung und dem mindestens einen Durchgangsloch von der Außenseite des Gehäuses her entfernt werden kann.

2. Inhalator nach Anspruch 1, dadurch gekennzeichnet, daß die mindestens zwei Teile des Gehäuses als geformtes Oberteil (1) und im allgemeinen flaches Unterteil (2) mit der Vertiefung für das Pulver ausgebildet sind, wobei diese Ober- und Unterteile (1, 2) an den Längsseiten miteinander verbunden sind.

3. Inhalator nach Anspruch 1 oder 2, dadurch gekennzeichnet, daß das Oberteil (1) aus einer Folie aus Kunststoffmaterial gegossen ist.

4. Inhalator nach Anspruch 3, dadurch gekennzeichnet, daß die Verengung (9) als Mulde in der Oberseite des Oberteils (1) gebildet ist, die sich quer zur Längserstreckung des röhrenförmigen Gehäuses orientiert und oberhalb der Vertiefung (3) im Unterteil des Gehäuses angeordnet ist.

5. Inhalator nach einem der vorhergehenden Ansprüche, dadurch gekennzeichnet, daß das Unterteil (2) aus mit Kunststoff laminierter Aluminiumfolie besteht.

6. Inhalator nach einem der vorhergehenden Ansprüche, dadurch gekennzeichnet, daß die Folie (6) die Form eines Bands aufweist, bei dem sich ein freies Ende durch den Lufteinlaß (4) nach draußen erstreckt, wobei das Band an den Rändern der Vertiefung (3) mit relativ schwachen Schweißverbindungen (22) befestigt ist.

7. Inhalator nach Anspruch 6, dadurch gekennzeichnet, daß das innere Ende der Folie (6) am Unterteil (2) zwischen dem Lufteinlaß (4) und der Vertiefung (3) befestigt ist, wobei sich die Folie an der Vertiefung (3) vorbei erstreckt und um diese herum befestigt ist und danach nach hinten umgebogen wird, um sich durch den Lufteinlaß (4) nach draußen zu erstrecken.

8. Inhalator nach Anspruch 7, dadurch gekennzeichnet, daß die schwachen Schweißverbindungen (22) einen stromabwärts weisenden Punkt bilden.

9. Inhalator nach einem der Ansprüche 6 bis 8, dadurch gekennzeichnet, daß sich Vorsprünge (7, 8) vom Oberteil (1) zwischen der Vertiefung (3) und dem Lufteinlaß (4) nach unten erstrecken, um die Folie (6) gegen das Unterteil (2) des Gehäuses zu halten.

10. Inhalator nach einem der vorhergehenden Ansprüche, dadurch gekennzeichnet, daß aggregationshemmende Mittel (11, 12, 13, 14, 15, 16) im Luftströmungsweg zwischen der Vertiefung (3) und dem Luftauslaß (5) angeordnet sind.

11. Inhalator nach Anspruch 10, dadurch gekennzeichnet, daß diese aggregationshemmenden Mittel (11, 12, 13, 14, 15, 16) planare, im Winkel von 20° bis 40°, vorzugsweise ca. 30°, relativ zur Längsrichtung des röhrenförmigen Gehäuses orientierte Oberflächen aufweisen, wobei jene Oberflächen im allgemeinen lotrecht zu einer Ebene durch die Längsachse des röhrenförmigen Gehäuses angeordnet sind und wobei bei Projektion der planaren Oberflächen auf einen Querschnitt des Gehäuses dieser Querschnitt im wesentlichen abgedeckt wird.

## Revendications

1. Inhalateur jetable actionné par la respiration, comprenant une enveloppe tubulaire comportant deux parties (1, 2) formant un chemin de circulation d'air ouvert aux deux extrémités, une extrémité formant une entrée d'air (4) et une extrémité formant une sortie d'air (5), ladite enveloppe comprenant un compartiment (3) pour stocker une poudre pharmaceutique à inhaler, ladite enveloppe étant façonnée de façon à présenter un étranglement (9) adjacent au compartiment (3) afin d'obtenir un flux d'air turbulent au niveau de l'étranglement (9) lors de l'inhalation, soulevant ainsi la poudre hors du compartiment (3) et la mélangeant dans le flux d'air, dans lequel le compartiment est prévu sous la forme d'un creux (3) qui est en contact avec l'air ambiant via au moins un trou de passage (24) prévu dans le fond dudit creux, le creux (3) étant situé en position adjacente à l'entrée d'air (4), ledit creux (3) et le au moins un trou de passage (24) étant couverts par un film (6) scellant le creux et le au moins un trou de passage de manière étanche à l'air, ledit film pouvant être retiré du creux et du au moins un trou de passage depuis l'extérieur de l'enveloppe.

2. Inhalateur suivant la revendication 1, caractérisé en ce que les au moins deux parties de l'enveloppe ont la forme d'une partie moulée supérieure (1) et d'une partie inférieure (2) essentiellement plate, dans laquelle est prévue le creux destiné à la poudre, lesdites parties supérieure et inférieure (1, 2) étant réunies le long de leurs côtés longitudinaux.

3. Inhalateur suivant la revendication 1 ou 2, caractérisé en ce que la partie supérieure (1) est moulée à partir d'une feuille de matière plastique.

4. Inhalateur suivant la revendication 3, caractérisé en ce que ledit étranglement (9) est réalisé sous la forme d'un renforcement dans la face supérieure de ladite partie supérieure (1), qui est orienté transversalement par rapport à l'étendue longitudinale de l'enveloppe tubulaire et est situé au-dessus du creux (3) formé dans la partie inférieure de l'enveloppe.

5. Inhalateur suivant l'une quelconque des revendications précédentes, caractérisé en ce que la partie inférieure (2) est fabriquée à partir d'un film d'aluminium stratifié avec des plastiques.

6. Inhalateur suivant l'une quelconque des revendications précédentes, caractérisé en ce que le film (6) a la forme d'un ruban ayant une extrémité libre s'étendant vers l'extérieur à travers l'entrée d'air (4), ledit ruban étant attaché autour des bords du creux (3) au moyen de soudures relativement faibles (22).

7. Inhalateur suivant la revendication 6, caractérisé en ce que l'extrémité intérieure du film (6) est attachée à la partie inférieure (2) entre l'entrée d'air (4) et le creux (3), le film s'étendant au-delà, et étant attaché autour, du creux (3), le film étant ensuite replié vers l'arrière de façon à s'étendre vers l'extérieur à travers l'entrée d'air (4).

8. Inhalateur suivant la revendication 7, caractérisé en ce que les soudures faibles (22) forment un point faisant face à l'aval.

9. Inhalateur suivant l'une quelconque des revendications 6 à 8, caractérisé en ce que des saillies (7, 8) s'étendent vers le bas depuis la partie supérieure (1) entre le creux (3) et l'entrée d'air (4) afin de maintenir le film (6) contre la partie inférieure (2) de l'enveloppe.

10. Inhalateur suivant l'une quelconque des revendications précédentes, caractérisé en ce que des moyens de partition (11, 12, 13, 14, 15, 16) sont situés dans le chemin de circulation d'air entre le creux (3) et la sortie d'air (5).

11. Inhalateur suivant la revendication 10, caractérisé en ce que lesdits moyens de partition (11, 12, 13, 14, 15, 16) comprennent des surfaces planes orientées à 20°-40°, de préférence à environ 30°, par rapport à la direction longitudinale de l'enveloppe tubulaire, lesdites surfaces étant disposées de manière sensiblement perpendiculaire à un plan passant par l'axe longitudinal de l'enveloppe tubulaire, une projection des surfaces planes sur une section transversale de l'enveloppe recouvrant sensiblement ladite section transversale.
